# EUROPEAN PATENT APPLICATION

(11) **EP 2 881 167 A1**
(43) Date of publication of application: **10.06.2015**
(21) Application number: 13826246.4
(22) Date of filing: 24.07.2013
(51) Int. Cl.: B01F 5/06, A61B 19/00, B01D 63/02, B01F 3/04, B08B 3/10

(54) **LIQUID SUPPLY DEVICE AND BIOLOGICAL CLEANING DEVICE**

(30) Priority: 28.07.2012 JP 2012167833
(71) Applicant: Ohdaira, Takeshi, Fukuoka-shi, Fukuoka 812-0054 (JP); Kyocera Medical Corporation, Osaka-shi, Osaka 532-0003 (JP)
(72) Inventor: OHDAIRA, Takeshi, Fukuoka-shi Fukuoka 812-0054 (JP); YAMADA, Masayuki, Kawasaki-shi Kanagawa 213-0012 (JP); KUWATA, Yasuaki, Kawasaki-shi Kanagawa 213-0012 (JP); TSURUMARU, Sayaka, Kawasaki-shi Kanagawa 213-0012 (JP); TANIZAKI, Yoshie, Toyohashi-shi Aichi 440-8601 (JP)
(74) Representative: Von Kreisler Selting Werner - Partnerschaft von Patentanwälten und Rechtsanwälten mbB
(86) International application number: PCT/JP2013/070041
(87) International publication number: WO 2014/021165

(57) **Abstract**

The purpose of the present invention is to provide a liquid supply device and biological cleaning device that, by supplying a sterile liquid including low-flow, low-pressure microbubbles, can dramatically increase cleaning effects and clean quickly and neatly without damaging a biological tissue. A liquid cleaning device (3), having a supply flow path (2) for supplying a liquid including microbubbles to a cleaning instrument (1) for cleaning an organism and to a cleaning target such as a medical instrument, is characterized by being provided with a tube pump (5) for delivering the liquid to the supply flow path (2) and a microbubble generator (B) that is provided midway on the supply flow path (2) and is for causing microbubbles to be generated in the liquid.

## Description

### TECHNICAL FIELD

The present invention relates to a liquid supply device suitable as a device that supplies a liquid including microscopic bubbles to a cleaning object such as a medical instrument with microscopic holes or a biological body and also relates to a biological cleaning device including the same.

### BACKGROUND ART

Recently, a study for microscopic bubbles such as microbubbles or nanobubbles has been carried out, and a microscopic bubble usage method or a microscopic bubble generator has been developed in various ways.

For example, Patent Document 1 discloses a method of using nano-bubbles and an apparatus for generating the nano-bubbles. Patent Document 1 discloses a method of using nano-bubbles by using characteristics, such as, sterilizing action, and surfactant action by the decrease in buoyancy caused by nano-bubbles, the increase in surface area, the increase in surface activity, the formation of local high-pressure field, and the realization of electrostatic polarization.

In detail, by relating these characteristics and actions to each other, the nano-bubbles exhibit an absorbing effect of dust ingredients (a detaching effect of dust ingredients), a high-speed washing effect on the surface of object, and a sterilizing effect, for various objects. In this way, various objects can be washed with low environmental load, thereby purifying contaminated water. In addition, it is described that the nano-bubbles can be applied into a living body, and thus, can be used for fatigue recovery.

Patent Document 2 discloses the technique of an anus washing apparatus including a nano-bubbles generator that generates nano-bubbles in liquid and a nozzle that spouts a fluid including the nano-bubbles generated by the nano-bubbles generator. It is described that since the nano-bubbles have an ultrafine form, when an anus is washed with the liquid including the nano-bubbles, the nano-bubbles move in an anus and permeate a rectum region, and thereby, the sterilizing action and washing action in those regions are performed.

Patent Document 3 discloses the technique for installing, at the upper part of a pressure-adjusting tank, a micro-bubbles-generating nozzle for collecting the bubbles with large size in the upper space of the inside of the tank by installing the pressure-adjusting tank in a circuit in a micro-bubbles generator, and thus, float-separating the bubbles with large size, and also, for restoring gas as the micro-bubbles in liquid by aspirating the gas from the upper space thereof.

### CITATION LIST

### PATENT DOCUMENT

Patent Document 1: JP 2004-121962 A
Patent Document 2: JP 2008-291521 A
Patent Document 3: JP 2011-206689 A

### DISCLOSURE OF THE INVENTION

### PROBLEM TO BE SOLVED BY THE INVENTION

Incidentally, when a body fluid or a foreign body in a living body, which is generated in a affected area and the surrounding area of the affected area for an operation or treatment, should be removed, it is preferable to remove rapidly and cleanly them. As one of the removal methods, a method is supposed which cleans a cleaning object by using a liquid including microscopic bubbles such as microbubbles.

However, Patent Document 1, Patent Document 2, and Patent Document 3 do not disclose the applications of the washing technique using the liquid including microscopic bubbles such as microbubbles or nanobubbles for removing a body fluid or a foreign body in a living body. Accordingly, the techniques disclosed in Patent Documents may not be directly employed for removing a body fluid or a foreign body in a living body.

The reason is that a high degree of effort is variously required, such that the extremely sensitive biological tissues are not damaged. In detail, the cleaning instrument capable of washing rapidly and cleanly by making the liquid without damaging the biological tissues and also by increasing the washing effect is required.

Further, when the diseased part or the vicinity thereof is cleaned, there is a need to consider the particle size distribution of microscopic bubbles and the flow rate and the discharge pressure of the liquid (the cleaning liquid) including sterile microscopic bubbles so as not to damage the biological tissue.

In addition, in a biological cleaning device which uses a liquid including microscopic bubbles such as microbubbles, there is a need to prevent the discharge of the polluted gas or the reverse flow of the liquid due to the back pressure of the bubble water.

Such problems are not limited to a brain surgery, and also exist in a general surgery that needs a cleaning process inside an abdominal cavity, a body cavity, and an intestinal tract.

Further, as the existing microbubble generator, a high-pressure melting type, a gas/liquid flow swirl type, and a porous type are developed. However, in any method, a problem arises in that any one of the water pressure and the air pressure increases in addition to the problem in which the water flow amount is too large.

Accordingly, an object of the invention is to provide a liquid supply device and a biological cleaning device capable of supplying a liquid containing microbubbles at a low flow rate and a low pressure and promptly and neatly cleaning a cleaning object by drastically improving a cleaning effect without damaging a biological tissue.

Further, an object of the invention is to provide a liquid supply device and a biological cleaning device capable of preventing the discharge of the polluted gas or the reverse flow of the liquid due to the back pressure of the bubble water.

### MEANS FOR SOLVING PROBLEM

In order to solve the above-described problems, there is provided a liquid supply device having a supply flow path which supplies a liquid containing microbubbles to a cleaning object, which includes a tube pump which feeds a liquid to the supply flow path and a microbubble generator which is provided in the course of the supply flow path and generates microbubbles in the liquid.

Since the liquid supply device of the invention includes the supply flow path which supplies the liquid containing microbubbles to the cleaning object, the tube pump which feeds the liquid to the supply flow path, and the microbubble generator which is provided in the course of the supply flow path and generates microbubbles in the liquid, it is possible to promptly supply the liquid containing microbubbles at a low flow rate and a low pressure in a sterile state by drastically improving the cleaning effect without damaging the biological tissue.

Further, since the tube pump which feeds the liquid to the supply flow path is employed, it is possible to feed the liquid to the supply flow path at a low flow rate and a low pressure suitable for cleaning the biological body. Further, even when the liquid is sterile water, it is possible to feed the liquid to the supply flow path in the sterile state, and to reliably prevent the discharge of the polluted gas or the reverse flow of the liquid due to the back pressure of the bubble water.

In the preferred embodiment of the invention, the microbubble generator includes an air supply membrane module which generates sterile microscopic bubbles in a liquid through a hollow fiber membrane, an air supply unit which supplies a pressurization gas to the air supply membrane module, and a shear stress generation nozzle which corresponds to a venture tube or a hole generating microbubbles in the liquid by causing the liquid including microscopic bubbles passing through the air supply membrane module to pass therethrough.

In this way, since the microbubble generator includes the air supply unit which supplies a pressurization gas to the air supply membrane module and the shear stress generation nozzle which generates microbubbles in the liquid by causing the liquid including microscopic bubbles passing through the air supply membrane module to pass therethrough, it is possible to generate microbubbles in the liquid flowing through the supply flow path at a low flow rate and a low pressure.

In the preferred embodiment of the invention, the air supply membrane module includes a plurality of hollow fiber membranes which include hollow portions causing the liquid to pass therethrough and causes only a gas to pass through the hollow fiber membranes in the thickness direction and a pressurization chamber which injects a gas into the liquid inside the hollow portion from the outside of the hollow fiber membrane.

Since the air supply membrane module has such a configuration, microscopic bubbles may be efficiently included in the liquid flowing through the hollow fiber membrane at a low flow rate and a low pressure. Further, the bacteria of the external air may be removed by the hollow fiber membrane, and hence the bubble water may be maintained in a sterile state.

In the preferred embodiment of the invention, the air supply membrane module causes air or at least one of a carbon gas, a nitrogen gas, an oxygen gas, and an ozone gas to selectively permeate the air supply membrane module to generate microscopic bubbles.

When the biological body is cleaned by using the liquid including microscopic bubbles generated by such a gas, it is possible to remedy a symptom such as TAO (thromboangiitis obliterans) or ASO (arteriosclerosis obliterans) by the heat retaining property of carbonated water to be mixed, to prevent a chemical injury of a tissue during a cleaning process using a bioinert nitrogen gas, or to improve a sterilization effect for a cleaning part by an ozone gas. Further, it is possible to activate a vascular endothelial growth factor in a blood vessel used to form a vascular vessel or to form a new blood vessel.

In the preferred embodiment of the invention, a bubble circulation container which includes a circulation path circulating a liquid containing microbubbles is disposed at the rear stage of the microbubble generator.

In this way, since the bubble circulation container which includes the circulation path circulating the liquid containing microbubbles is disposed at the rear stage of the microbubble generator, it is possible to continuously generate a liquid containing microbubbles regardless of whether the liquid containing microbubbles is used (for a cleaning process) and hence to maintain the quality thereof. Accordingly, it is possible to continuously or intermittently use the liquid containing microbubbles so as to clean the biological body during a surgery at any time.

In the preferred embodiment of the invention, a cleaning tube pump which supplies a liquid containing microbubbles to the cleaning object is disposed at the rear stage of the bubble circulation container.

In this way, when the cleaning tube pump which supplies the liquid containing microbubbles to the cleaning object is disposed at the rear stage of the bubble circulation container, it is possible to control the discharge amount of the liquid just by the control of the cleaning tube pump, and hence to simplify the control system and the liquid supply system when the biological body is cleaned while the entire function of the liquid supply device is maintained.

In the preferred embodiment of the invention, the cleaning tube pump includes an inlet for a liquid containing microbubbles inside the bubble circulation container.

In this way, since the cleaning tube pump includes the inlet for the liquid containing microbubbles inside the bubble circulation container, it is possible to supply the high-quality liquid containing microbubbles inside the bubble circulation container to the cleaning instrument.

In the preferred embodiment of the invention, a plurality of the shear stress generation nozzles of the microbubble generator are disposed in series or in parallel as a plurality of stages.

In this way, when the shear stress generation nozzles are disposed in series or in parallel as a plurality of stages, it is possible to promptly generate sterile water containing microbubbles having a more uniform particle diameter by the action of the shear stress generation nozzles of the plurality of stages. Accordingly, there is no need to employ a configuration in which the bubble circulation container and the circulation path are provided and the sterile water passes through the shear stress generation nozzle again.

In the preferred embodiment of the invention, the bubble circulation container is disposed above the cleaning object, and a natural drop tube for a liquid containing microbubbles extends from the bubble circulation container.

In this way, when the natural drop tube for the liquid containing microbubbles extends from the bubble circulation container disposed above the cleaning object, it is possible to adjust the discharge pressure of the liquid containing microbubbles just by changing the height position of the bubble circulation container. Accordingly, it is possible to set the discharge pressure of the liquid containing microbubbles to the discharge pressure suitable for the cleaning object.

In the preferred embodiment of the invention, the cleaning object is a medical instrument including microscopic holes. In this way, even when the medical instrument including the microscopic holes is a cleaning object, it is possible to promptly clean the cleaning object by the liquid containing microbubbles in a sterile state.

A biological cleaning device according to the invention includes a cleaning instrument which includes a discharge hole for a liquid cleaning a biological body and the above-described liquid supply device.

According to the biological cleaning device with such a configuration, since the cleaning object may be cleaned while the liquid containing microbubbles is supplied at a low flow rate and a low pressure and is discharged from the discharge hole of the cleaning instrument, it is possible to promptly and neatly clean the cleaning object by drastically improving the cleaning effect without damaging the biological tissue. Further, since the tube pump is employed, it is possible to prevent the discharge of the polluted gas or the reverse flow of the liquid due to the back pressure of the bubble water.

In the preferred embodiment of the invention, the cleaning instrument is directly connected to the shear stress generation nozzle of the rearmost stage among the plurality of shear generation nozzles through the supply flow path for the liquid containing microbubbles.

In this way, since the cleaning instrument is directly connected to the shear stress generation nozzle of the rearmost stage through the supply flow path, it is possible to further simplify the biological cleaning device including the control system by omitting the bubble circulation container or the cleaning tube pump.

In the preferred embodiment of the invention, the cleaning instrument is provided with an operation unit which controls the operation of the tube pump or the cleaning tube pump.

When the cleaning instrument is provided with the operation unit which controls the operation of the tube pump or the cleaning tube pump, it is possible to easily and conveniently perform a control involved with the discharge amount, the discharge stop operation, or the discharge start operation for the liquid containing microbubbles from the discharge hole of the cleaning instrument during a surgery.

### EFFECT OF THE INVENTION

An excellent effect may be obtained as below according to the invention. Since the cleaning object may be cleaned while the liquid containing microbubbles is supplied to the cleaning object at a low flow rate and a low pressure, it is possible to promptly clean the cleaning object in a sterile state while drastically improving the cleaning effect without damaging the biological tissue. Further, since the tube pump is employed, it is possible to prevent the discharge of the polluted gas or the reverse flow of the liquid due to the back pressure of the bubble water.

### BRIEF DESCRIPTION OF DRAWINGS

Fig. 1 is a schematic configuration diagram illustrating a liquid supply device according to a first embodiment of the invention;
Fig. 2 is a diagram illustrating a gas permeation principle of a hollow fiber membrane of the liquid supply device according to the first embodiment of the invention;
Fig. 3 is a partially cross-sectional front view illustrating an air supply membrane module that uses a hollow fiber membrane of the liquid supply device according to the first embodiment of the invention;
Fig. 4 is a graph illustrating a particle size distribution measurement result of sterile water containing microbubbles generated by the liquid supply device according to the first embodiment of the invention;
Fig. 5 is a schematic configuration diagram illustrating a liquid supply device according to a second embodiment of the invention;
Fig. 6 is a schematic configuration diagram illustrating a liquid supply device according to a third embodiment of the invention; and
Fig. 7 is a schematic configuration diagram illustrating an air supply membrane module of a liquid supply device according to a fourth embodiment of the invention.

### MODE(S) FOR CARRYING OUT THE INVENTION

### <First Embodiment>

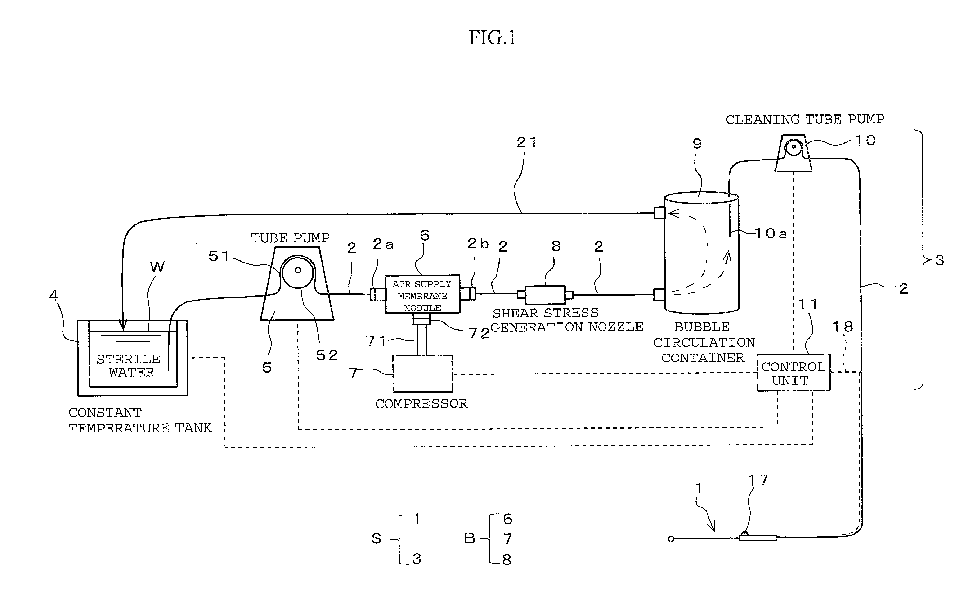

Hereinafter, a first embodiment of the invention will be described with reference to Figs. 1 to 4.

Fig. 1 illustrates an embodiment of a liquid supply device 3 that supplies a liquid at a low flow rate and a low pressure. The liquid supply device 3 includes a supply flow path 2 that supplies a liquid containing microbubbles to a cleaning instrument 1 used to clean a biological body. Accordingly, the cleaning instrument 1 and the liquid supply device 3 constitute a biological cleaning device S.

An example is illustrated in which the liquid supply device 3 according to the embodiment is suitable as a device that supplies a liquid for cleaning an intracranial hematoma in a brain surgery. Here, the microbubble may be a bubble having a bubble diameter of 1 to 200 µm and may also include a nanobubble having a bubble diameter smaller than 1 µm.

The liquid supply device 3 of the example illustrated in the drawing includes a constant temperature tank 4 which stores sterile water (liquid) w, a tube pump 5 which feeds the sterile water w inside the constant temperature tank 4 to the supply flow path 2, an air supply membrane module 6 which generates microscopic bubbles in the sterile water, a compressor (an air supply unit) 7 which supplies a pressurization gas to the air supply membrane module 6, a shear stress generation nozzle 8 which generates microbubbles by causing sterile water including microscopic bubbles to pass therethrough, a bubble circulation container 9 which includes a circulation path 21 to the constant temperature tank 4, a cleaning tube pump 10 which supplies sterile water to the cleaning instrument 1, and a control unit 11 which controls the above-described constituents. Next, the detailed configuration thereof will be described.

The constant temperature tank 4 is formed so as to store the sterile water w in a sterile state for a long time, and has a function of maintaining the sterile water w at a temperature (for example, about 37°C of a standard body temperature) desirable as a biological body cleaning purpose.

The tube pump 5 includes a tube body 51 which is elastic and serves as the supply flow path 2 for the sterile water w, a rotation roller 52, and a rotational driving unit (not illustrated) such as an electric motor, and has a function of causing the sterile water w inside the tube body 51 to flow to the downstream supply flow path 2 in a manner such that the rotation roller 52 rotates while sequentially pressing the tube body 51. Accordingly, the sterile water w may be supplied in a sterile state at a low flow rate and a low pressure. For example, the tube pump is set so as to exhibit a water feeding function of about a water pressure of 0.15 Mps and a flow rate of 1 liter/minute. Of course, the water feeding function may be set to the above-described value or more or or less.

The air supply membrane module 6 is provided in the course of the supply flow path 2 and is used to generate microscopic bubbles in the liquid through a hollow fiber membrane, and the compressor (the air supply unit) 7 is provided so as to supply a pressurization gas to the air supply membrane module 6. As the pressurization gas, oxygen, carbon dioxide, nitrogen, ozone, or the like is used if necessary other than air.

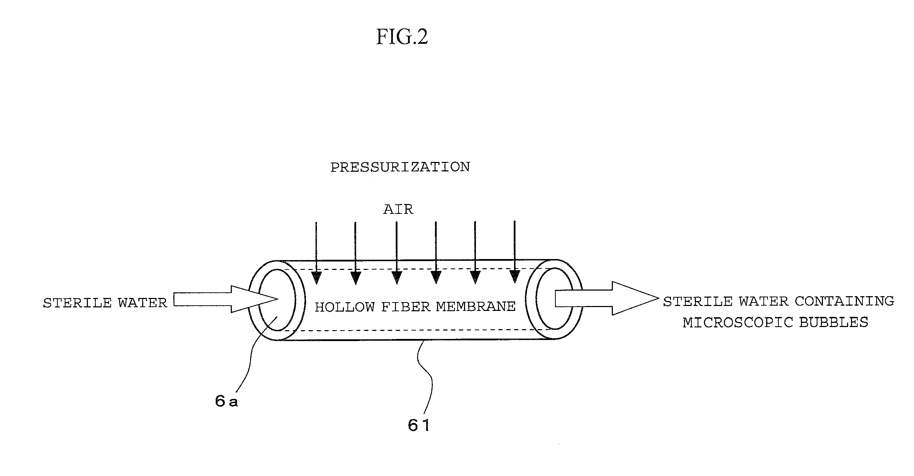

Fig. 2 is a diagram illustrating a gas permeation function of a hollow fiber membrane 61. As illustrated in this drawing, when a pressurization gas is fed from the outside of the hollow fiber membrane 61 while the sterile water w flows into the hollow portion 6a of the hollow fiber membrane 61, microscopic bubbles is generated in the sterile water through a microscopic structure formed in the membrane thickness direction of the hollow fiber membrane 61, and hence sterile water including microscopic bubbles is obtained. As the hollow fiber membrane 61, a single-layer porous membrane is generally used. Further, a triple-layer complex hollow fiber membrane having a structure in which a nonporous ultrathin membrane having highly selective gas permeability is sandwiched between porous layers in response to a bubble size or a gas used to generate bubbles.

As the highly selective permeable gas, a carbon gas, a nitrogen gas, an ozone gas, an oxygen gas, and the like may be exemplified other than air.

Furthermore, a method may be supposed in which microscopic bubbles to be mixed is generated by a carbon gas in order to remedy a symptom such as TAO (thromboangiitis obliterans) or ASO (arteriosclerosis obliterans) by the heat retaining property of carbonated water to be mixed, a method may be supposed in which a bioinert nitrogen gas is used as a gas to be mixed in order to prevent a chemical injury of a tissue during a cleaning process, or a method may be supposed in which a gas to be mixed is an ozone gas in order to improve a sterilization effect for a cleaning part.

Further, a method may be supposed in which a biological body is cleaned by using sterile water including microscopic bubbles generated by such a gas so as to activate "VGEF" (vascular endothelial growth factor) in a blood vessel used to form a vascular vessel or to form a new blood vessel.

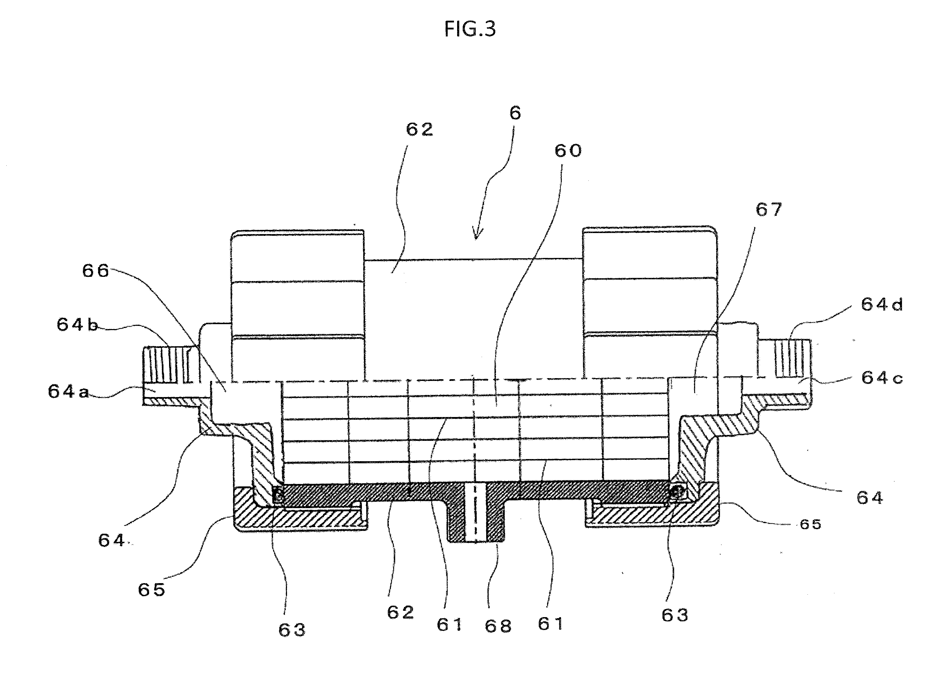

Fig. 3 is a partially cross-sectional front view illustrating the specific structure of the air supply membrane module 6 that uses a plurality of (several hundreds of) hollow fiber membranes 61. The air supply membrane module 6 includes a plurality of the hollow fiber membranes 61 with hollow portions 6a through which the sterile water w passes and a pressurization chamber 60 which injects a gas into the sterile water inside the hollow fiber membrane 61 from the outside of the hollow fiber membrane 61.

Specifically, the air supply membrane module 6 includes a tubular casing body 62, joints 64 and 64 which are disposed at both ends thereof through O-rings 63 and 63, and sealing covers 65 and 65 which seal the joints 64 while pressing the joints against the body 62. All hollow fiber membranes 61 are disposed inside the casing body 62. Each hollow fiber membrane 61 is disposed in the longitudinal direction of the casing body 62, one end side thereof communicates with a water supply space 66, and the other end side thereof communicates with a drainage space 67. Accordingly, the sterile water w which is supplied (injected) from the water supply port 64a becomes sterile water including microscopic bubbles when the sterile water passes through the hollow portion 6a of each hollow fiber membrane 61, and is fed to the shear stress generation nozzle 8 through a drainage port 64c and the supply flow path 2.

The one-end-side joint 64 including the water supply port 64a is provided with a male screw 64b used to connect the joint 64 to a joint 2a (see Fig. 1) of the end of the tube constituting the supply flow path 2. The other-end-side joint 64 including the drainage port 64c is provided with a male screw 64d used to connect the joint 64 to a joint 2b (see Fig. 1) of the end of the tube constituting the supply flow path 2 with respect to the shear stress generation nozzle 8.

The casing body 62 of the air supply membrane module 6 is provided with an intake joint 68 which is connected to a joint 72 of an air supply tube 71 of the compressor 7. Furthermore, any one of the joints is of a screw connection type.

In the embodiment, the hollow fiber membrane 61 of the air supply membrane module 6 is formed of polyethylene, the casing body 62 is formed of polycarbonate, and the O-ring is formed of silicon.

Furthermore, as the material of the hollow fiber membrane, a hydrophobic material may be used. For example, polypropylene, polyolefin such as 4-methyle-1-pentene, polyether, polymethylmethacrylate, polysulfone, polyacrylonitrile, fluororesin, or the like may be used.

Further, even the casing body may be formed of other resin materials such as acetal or polypropylene or metal.

The shear stress generation nozzle 8 is a nozzle which generates a shear stress in a venture tube or a hole (a thin flow path) used to generate microbubbles or nanobubbles smaller than microscopic bubbles, and has a function of generating microbubbles in a manner such that the sterile water including microscopic bubbles, obtained by causing the sterile water to pass through the air supply membrane module 6, passes through the nozzle.

The bubble circulation container 9 is disposed at the rear stage of the shear stress generation nozzle 8. The bubble circulation container 9 is provided with the circulation path 21 that circulates the sterile water w containing microbubbles obtained by the shear stress generation nozzle 8 to the constant temperature tank 4. When the water level of the sterile water w containing microbubbles and flowing into the bubble circulation container 9 through the shear stress generation nozzle 8 and the supply flow path 2 reaches a predetermined level, the sterile water is circulated to the constant temperature tank 4 through the circulation path 21 in design.

The cleaning tube pump 10 which supplies the sterile water w containing microbubbles to the cleaning instrument 1 is disposed at the rear stage of the bubble circulation container 9. As the cleaning tube pump 10, a small tube pump is employed. The cleaning tube pump 10 includes an inlet 10a used for a liquid containing microbubbles inside the bubble circulation container 9. Accordingly, the sterile water w containing microbubbles may be supplied to the cleaning instrument 1 at a low flow rate and a low pressure.

The control unit 11 is programmed so that the operation states of the constant temperature tank 4, the tube pump 5, the compressor 7, and the cleaning tube pump 10 are controlled so as to discharge the sterile water containing microbubbles suitable for cleaning the biological body from the cleaning instrument 1.

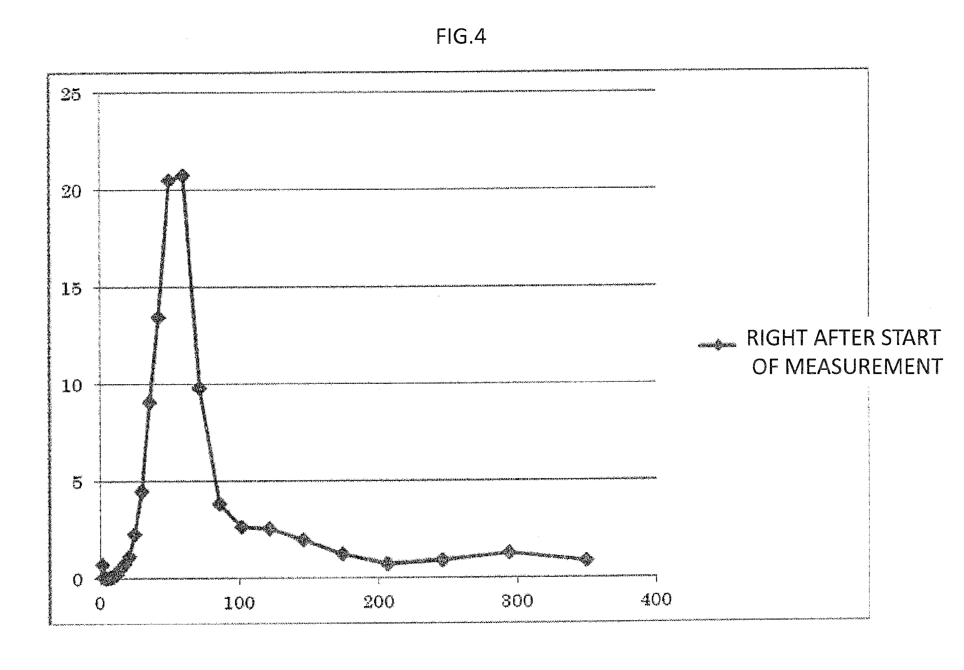

Further, the liquid supply device 3 is designed so as to have a particle size, a flow rate, and a discharge pressure in which the sterile water containing microbubbles does not damage the biological tissue. Specifically, a design is made in which a liquid including microscopic bubbles having a particle diameter (a bubble diameter) of 1 µm to 200 µm may be generated and desirably a liquid including many microbubbles of 10 µm to 100 µm may be generated as illustrated in Fig. 4.

Fig. 4 is a graph illustrating a particle size distribution measurement result of the sterile water w containing microbubbles generated by the liquid supply device 3.

In the embodiment, the above-described particle diameter is a particle diameter which is measured by using the measurement principle of laser diffractometry. As the laser diffraction-type particle size distribution measuring apparatus, a laser diffraction-type particle size distribution measuring apparatus, "HELOS&RODOS" manufactured by Sympatec Corporation.

As illustrated in Fig. 1, the cleaning instrument 1 includes a tubular portion which includes a discharge hole for the sterile water w containing microbubbles, a handle which is formed in the tubular portion, and an operation unit (an operation button) 17 which controls the operation of the cleaning tube pump 10. The operation button 17 is connected to the control unit 11 by a signal line 18 indicated by the dashed line of Fig. 1.

In the liquid supply device 3 of the embodiment, since the cleaning instrument 1 includes the supply flow path 2 which supplies the sterile water w containing microbubbles, the tube pump 5 which feeds the sterile water w to the supply flow path 2, and a microbubble generator B which is provided in the course of the supply flow path 2 and generates microbubbles in the sterile water w, it is possible to supply the sterile water containing microbubbles at a low flow rate and a low pressure and promptly and neatly cleaning a cleaning object by drastically improving a cleaning effect without damaging the biological tissue.

Further, since the tube pump 5 which feeds the sterile water to the supply flow path 2 is employed, it is possible to feed the sterile water to the supply flow path 2 at a low flow rate and a low pressure suitable for cleaning the biological body and to feed the sterile water to the supply flow path 2 in a sterile state. Further, since the tube pump 5 is employed, it is possible to reliably prevent the discharge of the polluted gas or the reverse flow of the liquid due to the back pressure of the bubble water.

Further, since the microbubble generator B includes the compressor 7 which supplies a pressurization gas to the air supply membrane module 6 and the shear stress generation nozzle 8 which generates microbubbles in the liquid by causing the liquid including microscopic bubbles and passing through the air supply membrane module 6 to pass therethrough, it is possible to generate microbubbles in the sterile water flowing inside the supply flow path 2 at a low flow rate and a low pressure.

Further, since the membrane used in the air supply membrane module 6 is formed so that air is supplied through a microscopic structure in a porous membrane and a triple-layer membrane, the intrusion of bacteria in the pressurization gas is prevented, and hence microbubbles may be generated while supplying a sterile gas.

Further, since the bubble circulation container 9 including the circulation path 21 used to circulate sterile water containing microbubbles is disposed at the rear stage of the shear stress generation nozzle 8, the sterile water containing microbubbles may be continuously generated regardless of whether the sterile water containing microbubbles is used (for a cleaning process), and hence the quality may be maintained. Accordingly, the sterile water containing microbubbles may be continuously or intermittently used as a biological cleaning liquid during a surgery at any time.

Further, when the cleaning tube pump 10 which supplies the sterile water w to the cleaning instrument 1 is disposed at the rear stage of the bubble circulation container 9, the discharge amount or the discharge pressure of the liquid may be controlled just by the control of the cleaning tube pump 10. Accordingly, it is possible to simplify the control system which cleans the biological body while the entire function of the liquid supply device 3 is maintained.

Further, according to the biological cleaning device S of the embodiment, an object may be cleaned in a manner such that the sterile water w containing microbubbles is supplied at a low flow rate and a low pressure and is discharged from the discharge hole of the cleaning instrument 1. For this reason, it is possible to promptly and neatly clean a cleaning object by drastically improving a cleaning effect without damaging the biological tissue. Further, since the tube pump is employed, it is possible to reliably prevent the discharge of the polluted gas or the reverse flow of the liquid due to the back pressure of the bubble water.

Further, since the cleaning instrument 1 is provided with the operation unit 17 which controls the operation of the cleaning tube pump 10, it is possible to easily and conveniently perform a control involved with the discharge amount, the discharge stop operation, or the discharge start operation for the sterile water containing microbubbles from the discharge hole of the cleaning instrument 1 during a surgery.

Furthermore, in the first embodiment, an example is illustrated in which the shear stress generation nozzle 8 of the bubble circulation container 9 is provided as one stage, but a configuration may be employed in which the shear stress generation nozzles are provided in series or two stages. In that case, it is possible to promptly obtain the sterile water containing microbubbles having a uniform particle diameter by the action of the shear stress generation nozzles 8 and 8 of two stages. Accordingly, the bubble circulation container 9 may be used as a storage tank for the sterile water containing microbubbles while the circulation path 21 is omitted.

### <Second Embodiment>

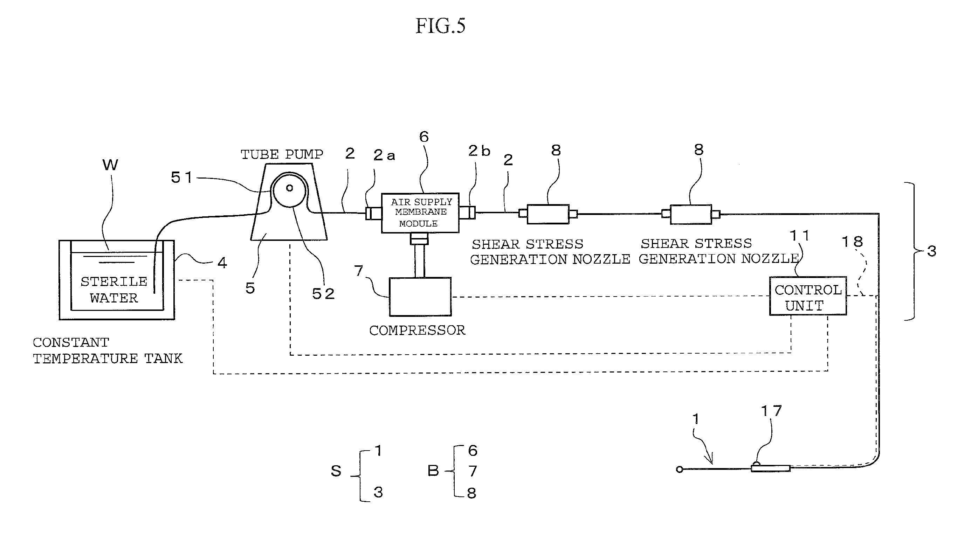

Fig. 5 is a schematic configuration diagram illustrating a second embodiment of the liquid supply device according to the invention. Furthermore, in the embodiment, the identical reference numerals will be given to the components basically identical to the above-described embodiment, and the description thereof will be made briefly.

As illustrated in Fig. 5, the liquid supply device S according to the second embodiment includes the constant temperature tank 4 which stores the sterile water (the liquid) w, the tube pump 5 which feeds the sterile water w inside the constant temperature tank 4 to the supply flow path 2, the air supply membrane module 6 which generates sterile microscopic bubbles in the sterile water, the compressor (the air supply unit) 7 which supplies a pressurization gas to the air supply membrane module 6, the shear stress generation nozzle 8 which generates microbubbles by causing the sterile water including microscopic bubbles to pass therethrough, and the control unit 11 which controls the above-described constituents.

However, in the second embodiment, the shear stress generation nozzles 8 are provided in series as two stages, and the second-stage shear stress generation nozzle 8 is directly connected to the cleaning instrument 1 through the supply flow path 2. Accordingly, in the embodiment, the bubble circulation container 9, the circulation path 21, and the cleaning tube pump 10 are not provided.

In this way, when the shear stress generation nozzles 8 are provided in series as two stages, it is possible to promptly obtain the sterile water containing microbubbles having a more uniform particle diameter by the action of two stages of the shear stress generation nozzles 8 and 8. For this reason, there is no need to employ a configuration in which the bubble circulation container 9 and the circulation path 21 are provided and the sterile water passes through the shear stress generation nozzle 8 again as in the first embodiment.

Further, since the cleaning instrument 1 is directly connected to the second-stage shear stress generation nozzle 8 through the supply flow path 2, it is possible to further simplify the biological cleaning device including the control system by omitting the bubble circulation container or the cleaning tube pump illustrated in the first embodiment.

Furthermore, in the second embodiment, an example is illustrated in the shear stress generation nozzles 8 are provided in series as two stages, but may be disposed as two stages or more if necessary. Further, the shear stress generation nozzles may be disposed in parallel as a plurality of stages.

### <Third Embodiment>

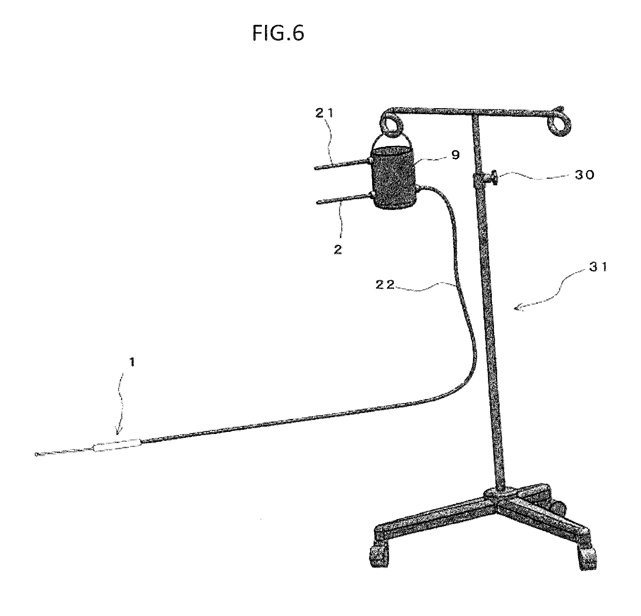

Fig. 6 is a schematic configuration diagram illustrating a main part of a third embodiment of the liquid supply device according to the invention. Furthermore, in the embodiment, the identical reference numerals will be given to the components basically identical to the above-described embodiment, and the description thereof will be made briefly.

As illustrated in Fig. 6, the liquid supply device S according to the third embodiment has a configuration in which the bubble circulation container 9 is suspended so that the sterile water w drops naturally by the own weight.

That is, a configuration is employed in which the bubble circulation container 9 is disposed above a cleaning object such as a biological body by using a suspending member 30 and a natural drop tube 22 for a liquid containing microbubbles extends from the bubble circulation container 9. The cleaning instrument 1 illustrated in Fig. 1 is connected to the front end (the free end) of the natural drop tube 22. Furthermore, the suspending member 30 is not particularly limited, but a configuration including a height adjustment instrument 31 is desirable.

In this way, when the natural drop tube 22 for the liquid containing microbubbles extends from the bubble circulation container 9 disposed above the cleaning object, the discharge pressure of the liquid containing microbubbles may be adjusted just by the adjustment of the height position of the bubble circulation container 9. Accordingly, the discharge pressure of the liquid including microbubbles may be set to a discharge pressure suitable for the cleaning object.

### <Fourth Embodiment>

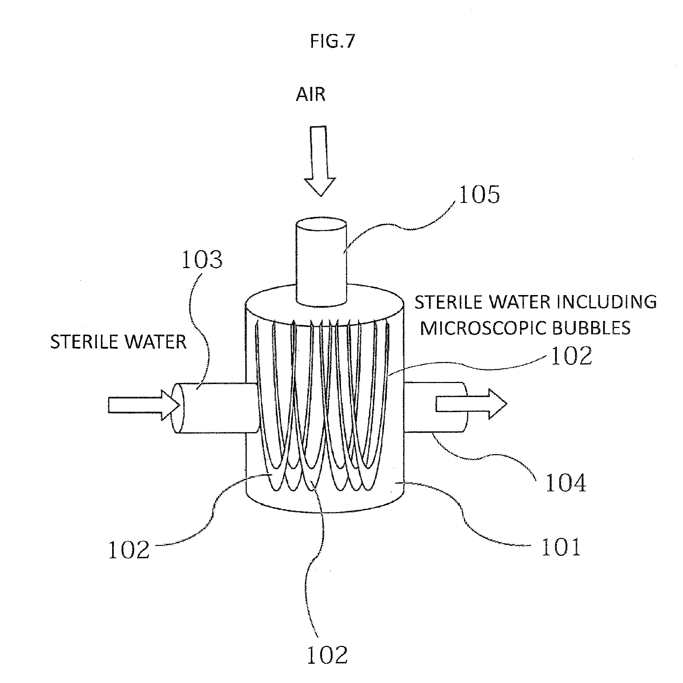

In the above-described embodiments, as illustrated in Fig. 2, the air supply membrane module 6 has a configuration in which microscopic bubbles are generated in the sterile water through the hollow fiber membrane 61 in a manner such that the pressurization gas is fed from the outside of the hollow fiber membrane 61 while the sterile water w flows into the hollow portion 6a of the hollow fiber membrane 61. However, for example, an air supply membrane module 100 having a configuration illustrated in Fig. 7 may be employed.

The air supply membrane module 100 includes a hollow columnar housing 101, a plurality of hollow fiber membranes 102 which are disposed inside the housing 101, an inflow port 103 and an outflow port 104 for the sterile water w provided in the outer peripheral surface of the housing 101, and a gas supply port 105. Accordingly, the air supply membrane module 100 generates microscopic bubbles in the sterile water by causing the sterile water to flow into the housing 101 and injecting a gas into the hollow portion of the hollow fiber membrane 102.

Each hollow fiber membrane 102 is bent in a U-shape in the example illustrated in the drawing, and the openings of both ends respectively connected to the gas supply port 105 in a communication state. Accordingly, the sterile water w which is supplied from the inflow port 103 becomes sterile water including microscopic bubbles when the sterile water passes through the housing 101, and is fed to the shear stress generation nozzle 8 through the outflow port 104 and the supply flow path 2.

In this way, when the sterile water flows into the housing 101 so as to inject a gas into the hollow portion of the hollow fiber membrane 102, it is possible to easily generate microscopic bubbles in the sterile water w even when the sterile water flows at a low flow rate and a low pressure, and hence to further decrease the size of the air supply membrane module.

### EXPLANATIONS OF LETTERS OR NUMERALS

1 Cleaning instrument
2 Supply flow path
21 Circulation path
22 Natural drop tube
3 Liquid supply device
30 Suspending member
31 Height adjustment instrument
4 Constant temperature tank
5 Tube pump
6, 100 Air supply membrane module
7 Compressor (air supply unit)
8 Shear stress generation nozzle
9 Bubble circulation container
10 Cleaning tube pump
11 Control unit
17 Operation button
w Sterile water (liquid)
B Microbubble generator
S Biological cleaning device

## Claims

1. A liquid supply device including a supply flow path supplying a sterile liquid containing microbubbles to a cleaning object, comprising:
a tube pump which feeds a liquid to the supply flow path; and
a microbubble generator which is provided in the course of the supply flow path and generates microbubbles in the liquid.

2. The liquid supply device according to claim 1,
wherein the microbubble generator includes an air supply membrane module which generates sterile microscopic bubbles in a liquid through a hollow fiber membrane, an air supply unit which supplies a pressurization gas to the air supply membrane module, and a shear stress generation nozzle which generates microbubbles in the liquid by causing the liquid including microscopic bubbles passing through the air supply membrane module to pass therethrough.

3. The liquid supply device according to claim 2,
wherein the air supply membrane module includes a plurality of hollow fiber membranes which include hollow portions causing the liquid to pass therethrough and cause only a gas to pass through the membrane in the thickness direction and a pressurization chamber which injects a gas from the outside into the liquid of the hollow portions of the hollow fiber membranes.

4. The liquid supply device according to claim 2 or 3,
wherein the air supply membrane module causes air or at least one of a carbon gas, a nitrogen gas, an oxygen gas, and an ozone gas to selectively permeate the air supply membrane module to generate microscopic bubbles.

5. The liquid supply device according to claim 2 or 3,
wherein the shear stress generation nozzle of the microbubble generator is disposed in series or in parallel as a plurality of stages.

6. The liquid supply device according to any one of claims 1 to 4,
wherein a bubble circulation container which includes a circulation path for circulating a liquid including microscopic bubbles is disposed at the rear stage of the microbubble generator.

7. The liquid supply device according to claim 6,
wherein a cleaning tube pump which supplies a liquid containing microbubbles to the cleaning object is disposed at the rear stage of the bubble circulation container.

8. The liquid supply device according to claim 7,
wherein the cleaning tube pump includes an inlet for a liquid containing microbubbles inside the bubble circulation container.

9. The liquid supply device according to any one of claims 6 to 8,
wherein the bubble circulation container is disposed above the cleaning object, and a natural drop tube for a liquid containing microbubbles extends from the bubble circulation container.

10. The liquid supply device according to any one of claims 1 to 9,
wherein the cleaning object is a medical instrument including a microscopic hole.

11. A biological cleaning device comprising:
a cleaning instrument which includes a discharge hole for a liquid containing microbubbles cleaning a biological body; and
the liquid supply device according to any one of claims 1 to 10.

12. The biological cleaning device according to claim 11,
wherein the cleaning instrument is directly connected to the shear stress generation nozzle of the rearmost stage among the plurality of shear generation nozzles through the supply flow path for the liquid containing microbubbles.

13. The biological cleaning device according to claim 11,
wherein the cleaning instrument is connected to the natural drop tube.

14. The biological cleaning device according to any one of claims 11 to 13,
wherein the cleaning instrument is provided with an operation unit which controls the operation of the tube pump or the cleaning tube pump.
